Europäisches Patentamt

European Patent Office

Office européen des brevets

(19)

(11) Publication number: **0 040 683**
**B1**

(12) **EUROPEAN PATENT SPECIFICATION**

(45) Date of publication of patent specification: **19.02.86**

(21) Application number: **81102258.1**

(22) Date of filing: **25.03.81**

(51) Int. Cl.⁴: **A 61 B 17/10**

(54) Surgical stapling instrument.

(30) Priority: **27.05.80 US 153228**
**27.05.80 US 153229**

(43) Date of publication of application:
**02.12.81 Bulletin 81/48**

(45) Publication of the grant of the patent:
**19.02.86 Bulletin 86/08**

(84) Designated Contracting States:
**BE CH DE FR GB IT LI NL SE**

(56) References cited:
**DE-C- 819 088**
**GB-A- 939 929**
**US-A-1 452 373**
**US-A-2 275 548**
**US-A-2 660 725**
**US-A-3 510 043**
**US-A-3 873 016**
**US-A-4 043 504**
**US-A-4 179 057**

(73) Proprietor: **AMERICAN CYANAMID COMPANY**
**1937 West Main Street P.O. Box 60**
**Stamford Connecticut 06904 (US)**

(72) Inventor: **Campbell, Jay E.**
**Lonely Cottage Drive**
**Upper Black Eddy Pennsylvania 18972 (US)**
Inventor: **Reichmann, Richard H.**
**141 Sherwood Drive**
**Churchville Pennsylvania 18966 (US)**
Inventor: **Li, Lehmann K.**
**305 Barlow Road**
**Fairfield Connecticut 06430 (US)**

(74) Representative: **Wächtershäuser, Günter, Dr.**
**Tal 29**
**D-8000 München 2 (DE)**

# Description

The invention relates to a stapling instrument comprising a handle, a trigger, a track for carrying at least one staple, a blade for pushing said staple against an anvil for closing said staple and a leaf spring located at the end of said track and retaining said staple prior to actuating the trigger.

Such a stapling instrument is known from US—A—4 043 504, whereby, however, the leaf spring serves for resiliently holding the proximal staple while said staple is laterally displaced into the longitudinal path of movement of said forming blade and thereafter pushed by said blade toward said anvil. Such a stapling instrument is mechanically complicated, involving additionally to the longitudinal feeding movement of the staples and the longitudinal movement of the proximal staple by said blade also a lateral displacement movement of the proximal staple.

US—A—3 873 016 describes a stapling instrument, whereby the forming blade moves perpendicular to the staple feeding movement. However, the proximal staple is rather loosely held while being pushed by said forming blade towards said anvil, which reduces the reliability of the instrument.

Therefore the present invention is concerned with the problem of increasing the reliability of the stapling instrument.

This problem is solved by the features of the main claim. Further preferred embodiments are characterized in the sub-claims.

The stapling instrument is preferably a surgical skin/fascia stapling instrument. The stapling instrument characterized in the main claim and especially the preferred embodiments characterized in the sub-claims show the following characteristics and advantages.

One advantage is the relative size of the instrument which is maintained while the number of mechanical parts is decreased. Therefore, as a general statement, the ease of operation and the reliability of the instrument is improved. The operation and reliability of an instrument can be critical in the surgical operating area where even seconds may determine if a surgical operation or procedure is a success. Another advantage is the orientation of the flange on the anvil surface. The flange orientation is opposite to the direction of stapling. For most surgeons, the natural direction of stapling is always away from their body. The orientation of the flange allows the surgeon to advance the instrument and remove the formed staple in a direction away from the body. This has still another advantage in that while stapling the surgeon cannot accidentally brush against the open wound site.

Still another advantage of this instrument is the stapling indicator which is on top of the handle. The instrument thus does not have to be turned over to determine the number of staples remaining in the instrument. Still further, another advantage is the location of the trigger means which are internal to the handle. This seems to provide a more sterile environment and to prevent or decrease the likelihood of pinching a finger or fingers during use. Yet another advantage is the configuration of the instrument. In many surgical procedures, the instrument configuration may give a better field of vision of the wound site to the surgeon.

The surgical stapling control means of claims 3 and 4 also has advantages over the prior art. The control means prevents the trigger from returning to its initial position if the compression is interrupted. This, has the advantage of preventing a second staple from being formed on the anvil flange before a first staple is separated from the instrument. Another advantage of the control means is interrupted stapling. The surgeon can now stop the compression of the trigger into the handle to realign the instrument over the wound site. Thus, the possibility of a perfect stapling procedure is greatly enhanced. Finally, the control means are automatically disengaged on completely compressing the trigger. Thus, the control means do not have to be manually reset after a single staple is formed and separated from the instrument. Generally the surgical stapling instrument comprises a handle and a trigger pivotally attached and on compression internal to the handle. The forward portion of the handle contains a track; a plurality of staples loaded and staple advancing means carried on the track; a track cover mounted on the track; an anvil surface mounted and a first bias means movably mounted on the cover, the said anvil surface terminating in a perpendicular flange; a staple adjacent the anvil surface; and a retainer spring mounted on the anvil surface and separating the staple from the flange.

An alternative surgical stapling instrument comprises a handle and a trigger pivotally attached and on compression internal to said handle has also been invented. The forward portion of the handle contains: a track; a leaf spring on the terminal end of said track; a plurality of staples loaded and staple advancing means carried on said track; a track cover mounted on said track; an anvil surface mounted and a first bias means movably mounted on the cover, the said anvil surface terminating in a perpendicular flange; and a staple adjacent said anvil surface separated from said flange by said leaf spring.

In both instruments, the forward portion of the handle also contains a guide block which is mounted on the track cover adjacent the anvil surface.

In both instruments, a forming blade and a second bias means are movably mounted on the guide block, the forming blade and the trigger having coordinating surfaces.

In both instruments, on compressing the trigger into the handle, the forming blade pushes the staple downward, the staple displaces the leaf spring, the leaf spring moves back to its initial position and the staple forms on the flange. On releasing the trigger and advancing the instrument, the formed staple is separated from the flange, the second bias means returns the trigger and the forming blade to their initial positions, and the first bias means activates the staple advancing means to place the forwardmost staple adjacent the anvil surface.

In both instruments, the track cover can be adjacent the forward portion of the trigger and/or the forming blade can be located between the forward portion of the track cover and the anvil surface.

One embodiment of the stapling instrument or of the alternative stapling instrument described above comprises an indicator to indicate the number of staples remaining in the instrument. The indicator has an initial end visible in the handle. A terminal end is movably mounted on the staple advancing means such that on releasing the trigger, the first bias means pulls the terminal end and thus moves the initial end of the indicator.

The control means comprises a multi-toothed ratchet on the rearward position of the trigger; at least one guide pin attached to the initial end of the ratchet; a nonpivoting pawl attached to the rearward portion of the handle to coordinate with the ratchet; and guide means adjacent the rearward portion of the handle to coordinate with and provide tension to the guide pin.

An alternative control means comprises a multi-toothed ratchet on the rearward portion of the handle; a pawl attached to the rearward portion of the trigger to coordinate with the ratchet; at least one guide pin attached to the initial end of the pawl; and guide means adjacent the rearward portion of said handle to coordinate with and provide tension to the guide pin.

On partially compressing the trigger the guide means provide tension on the guide pin and the ratchet engages the pawl. On completely compressing the trigger, the guide pin crosses over the top of the guide means causing the ratchet to be disengaged from the pawl.

In one embodiment the guide means described above are two cams attached to each side of the handles. In another embodiment, the stapling control means comprises two guide pins to coordinate with the two cams.

In yet another embodiment, the staple forming means described above is a track; a plurality of staples loaded and staple advancing means carried on the track; a track cover mounted on the track; an anvil surface mounted and a first bias means movably mounted on the cover, the said anvil surface terminating in a perpendicular flange; a staple adjacent the anvil surface; a retainer spring mounted on the anvil surface or a leaf spring on the terminal end of the track, and separating the staple from the flange; a guide block mounted on the cover adjacent the anvil surface; and a forming blade and a second bias means movably mounted on the guide means, the forming blade and the trigger having coordinating surfaces. The trigger has "overtravel". That is on compressing the trigger into the handle the staple forms on the flange before the guide pins cross over the top of the guide means causing the ratchet to be disengaged from the pawl.

Description of the Drawings

Figures 1 and 2 are side and top views respectively of the stapling instrument;

Figure 3 is a broken perspective view of the front portion of the instrument shown in Figures 1 and 2;

Figures 4A and 4B are perspective views showing the parts of the stapling instrument control means in the handle and in the handle cover, respectively;

Figure 5 is a broken sectional view of the stapling instrument control means;

Figure 6 is a perspective view of the instrument trigger, and an expanded view of the track and track cover;

Figures 7 and 8 are sectional side, and top views respectively of the instrument track cover;

Figures 9 and 10 are sectional side, and top views respectively of the instrument track;

Figure 11 is an expanded view showing the relationship of the forming blade, bending anvil and retainer spring; and

Figures 12 and 13 are broken and sectional side views of Figure 1 showing the position and relationship of the staple and retainer spring in the initial position and on compressing the instrument trigger;

Figures 14 to 16 are broken rear views of the bending anvil showing the relationship of the staple, forming blade, retainer spring and anvil flange during compression of the instrument trigger;

Figure 17 is an expanded view showing the relationship of the forming blade, first bias means, guide block, track cover, bending anvil and retainer spring.

Figures 18A and 18B are perspective views showing an alternative embodiment of the stapling instrument control means;

Figure 19 is a broken sectional view of the alternative stapling instrument control means of Figure 18;

Figure 20 is a broken perspective view of the alternative control means in the trigger;

Figure 21 is an expanded view of an alternative embodiment of the track and track cover;

Figures 22 and 23 are broken sectional side and top views, respectively of the instrument track shown in Figure 21;

Figure 24 is an expanded view showing the relationship of the forming blade, bending anvil and leaf spring shown in Figure 21; and

Figures 25 to 27 are broken and sectional side views showing respectively the position and relationship of the staple and leaf spring in the initial position, on partial compressing and on complete compressing of the instrument trigger.

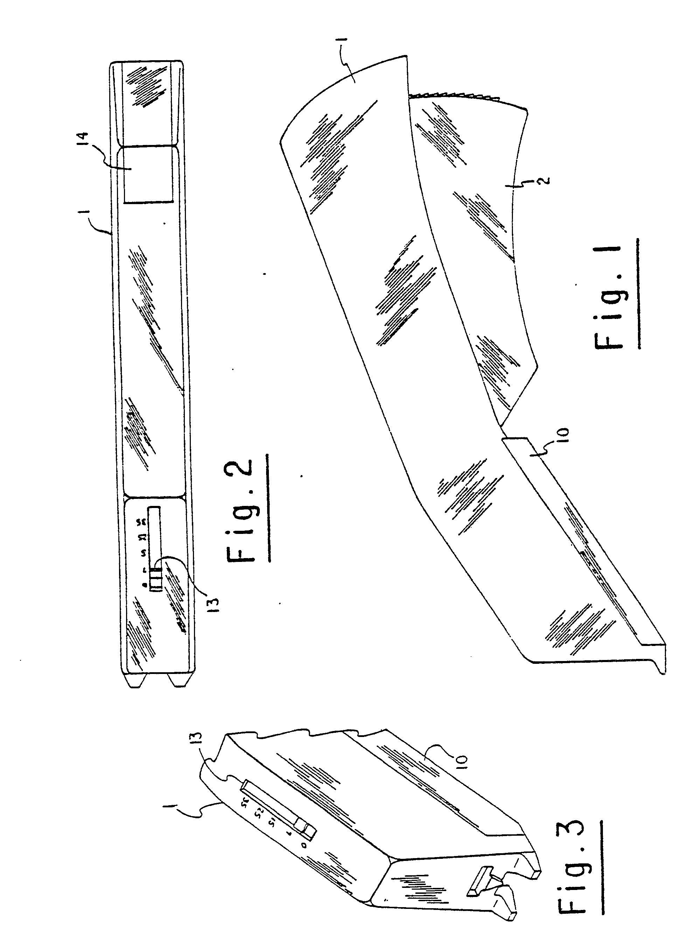

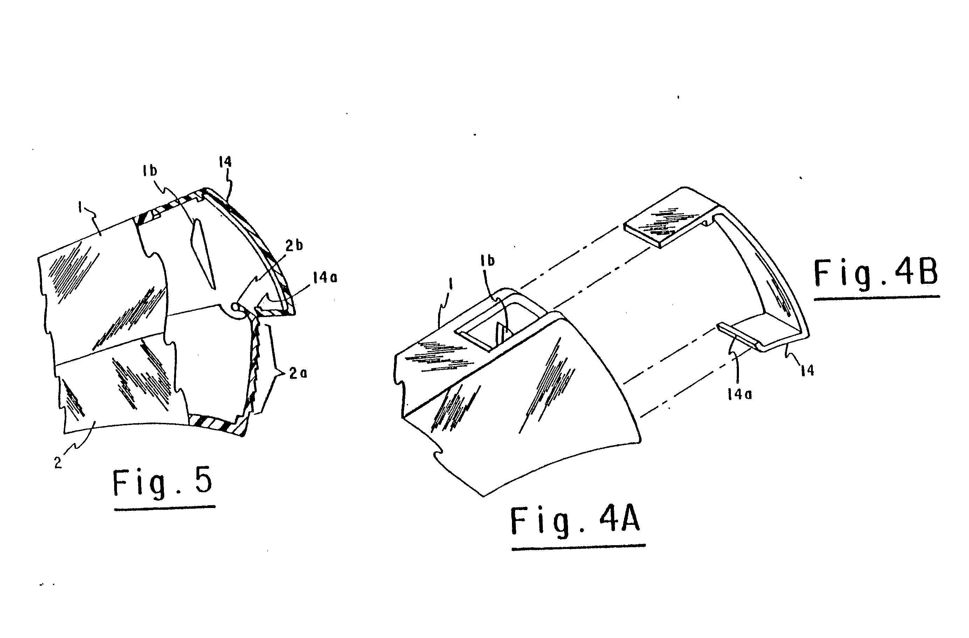

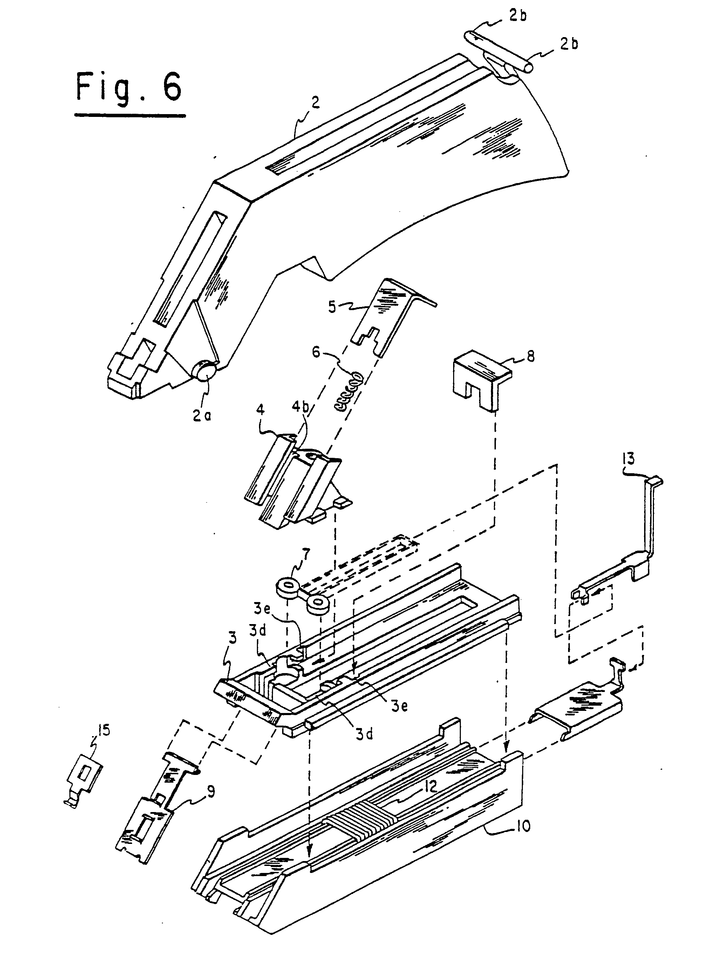

Referring to Figures 1 to 3, the instrument comprises a handle 1 and a trigger 2. A staple track 10 (more fully described in Figures 6 and 9 to 10) is inserted and attached to the forward portion of the handle 1, for example by cementing or sonic welding. The initial end of an indicator 13 is visible through an opening in the forward top portion of the handle 1. A combined ratchet stop and cover 14 (more fully described in Figures 4 and 5) is attached to the rear portion of handle 1.

Referring to Figures 6 to 11 and 17, the track cover 3 is assembled as follows. The first bias means 7, which preferably is a negator spring is mounted into the openings 3a in track cover 3. Retainer spring 15 is inserted onto bending anvil retainer tabs 9a. The bending anvil 9 and the retainer spring 15 are then placed through the opening 3b. The terminal end 13a of indicator 13 is mounted onto the track cover 3 in front of the spring 7 and protrudes through the bottom 3c of the track cover 3.

The guide block 4 is mounted under tabs 3d. A locking wedge 8 is then pushed into slots 3e to hold the guide block 4 on the track cover 3. Other means for holding the guide block on the track cover can be used, for example bonding, riveting, peening, tacking or welding.

The second bias means 6, preferably a spring, is inserted into the guide block opening 4a. The forming blade 5 is mounted through the guides 4b in the guide block. The vertical surface of the forming blade 5 is between the forward portion of the track cover 3 and the anvil surface 9.

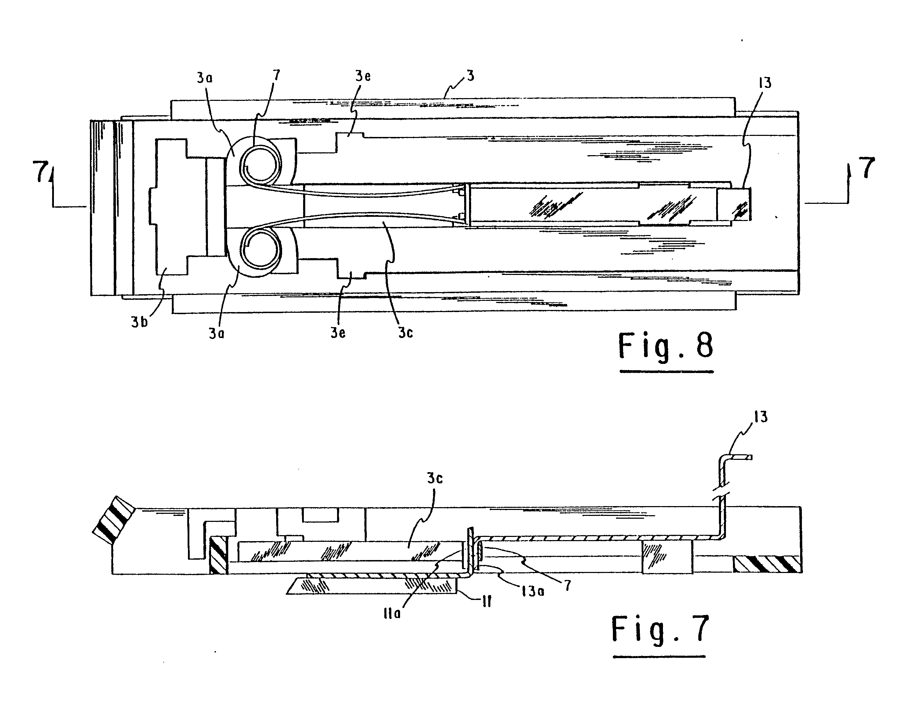

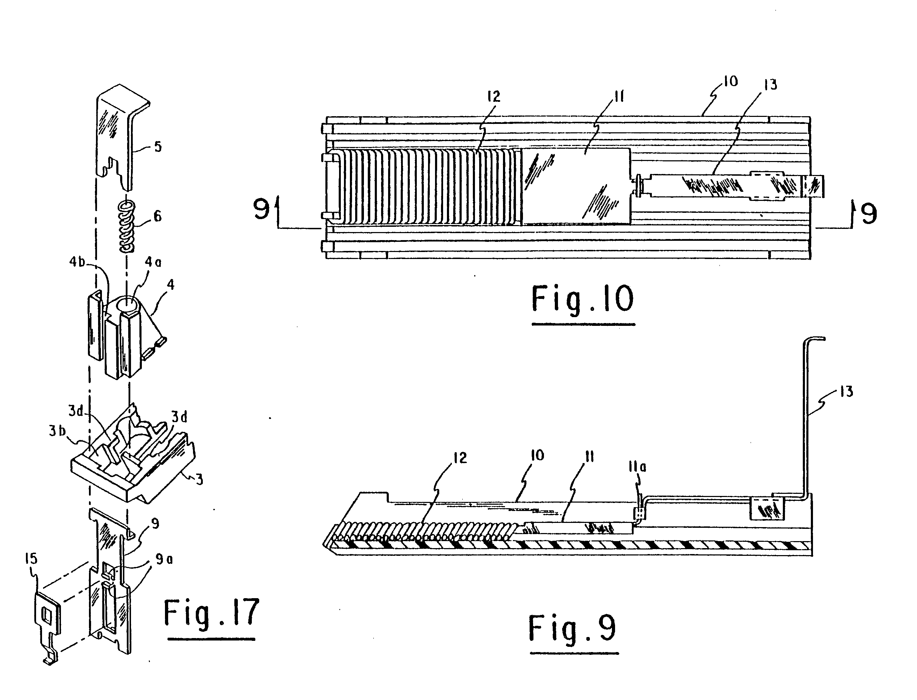

Referring specifically to Figures 9 and 10, staples 12 are loaded onto the track 10. The staple advancing means, preferably a staple pusher 11 is carried on the track 10 behind the staples 12 by the first bias means 7 (shown e.g., in Figure 8). The indicator 13 is carried with the staple pusher 11 by the first bias means 7. The staples 12 in Figures 9 and 10 are shown in their orientation when the instrument is in the position shown in Figure 1.

The track cover 3 is then mounted onto the track 10 for example by sonic welding. The spring 7 is then attached to the advancing means tab 11a by pulling back on the indicator 13 and engaging the center of the extended spring 7 with the advancing means tab 11a. The trigger pivots 2a are placed against stops in the forward portion of the handle 1. The track cover 3 and track 10 are then inserted and attached to the forward portion of the handle 1, for example by cementing or sonic welding. The trigger pivots 2a are thus captured.

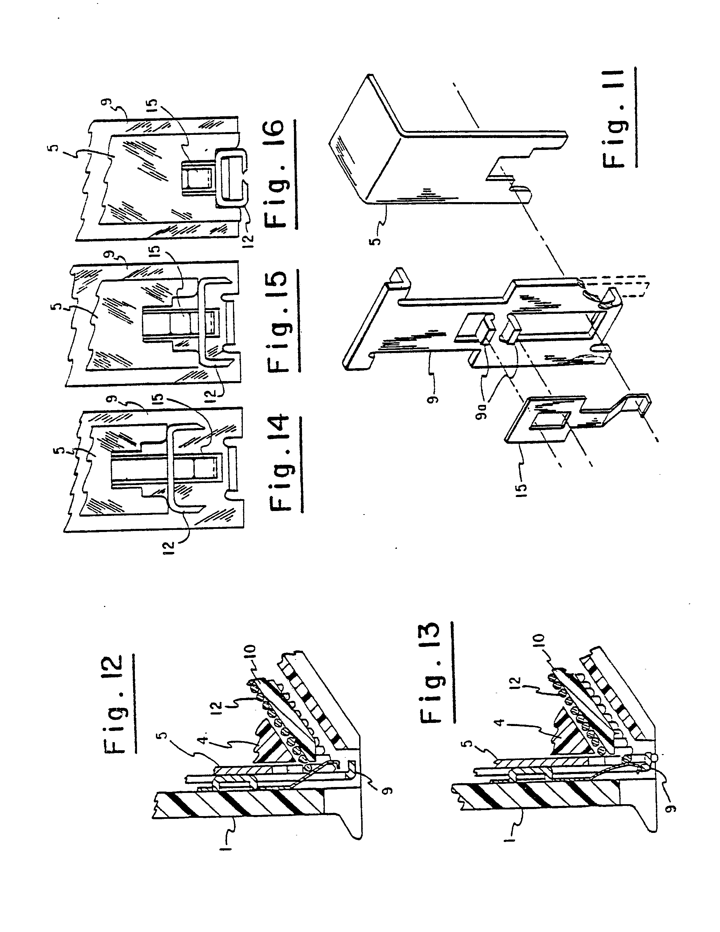

Referring to Figures 12 to 17, the stapling instrument is used by placing the anvil surface 9 adjacent a wound opening or between skin or fascia. The trigger 2 is then compressed into the handle 1 (shown in Figure 1). The front end of trigger 2 engages the top flange of forming blade 5, forcing it down through the guides 4b on guide block 4. The lower edges of the forming blade have a recessed area to engage staple 12. The staple is pushed downward and forced to bend at right angles on either side of the lower flange of anvil 9.

In the initial or rest position, the staple 12 is adjacent the vertical surface of the anvil 9, as shown in Figure 12. The forming blade 5 lowers and pushes the staple downward and onto the anvil flange. The forming of the staple around the anvil lower flange is well known in the prior art. By releasing the trigger and advancing the instrument, the staple 12 is separated from the anvil flange.

When releasing trigger 2, spring 6 returns forming blade 5 and trigger 2 to their relaxed positions. Spring 7 pulls against pusher 11 to advance the plurality of staples. Each time trigger 2 is compressed indicator 13 advances with pusher 11. An indication of the staple depletion appears in the opening in the top forward portion of handle 1.

Referring to Figures 4 and 5, to prevent partially compressing the trigger 2, partially forming a staple 12, and then allowing the trigger to return and pick up the next staple, a ratchet 2a is built into the rear of the trigger 2 and cam guides 1b into handle 1. When the trigger 2 is compressed, the ratchet 2a engages stop 14a and prevents the trigger from returning to its relaxed position. The trigger must be compressed past the last ratchet tooth 2a and must be completely closed so that the guide pins 2b (more fully shown in Figure 6) move up and cross over the cam guides 1b.

The guide pins are spring loaded. Thus when the trigger is completely compressed, the guide pins cross over the top of the cam guides 1b. On releasing the trigger from a final compression, the ratchet is thus prevented from locking on the stop 14a.

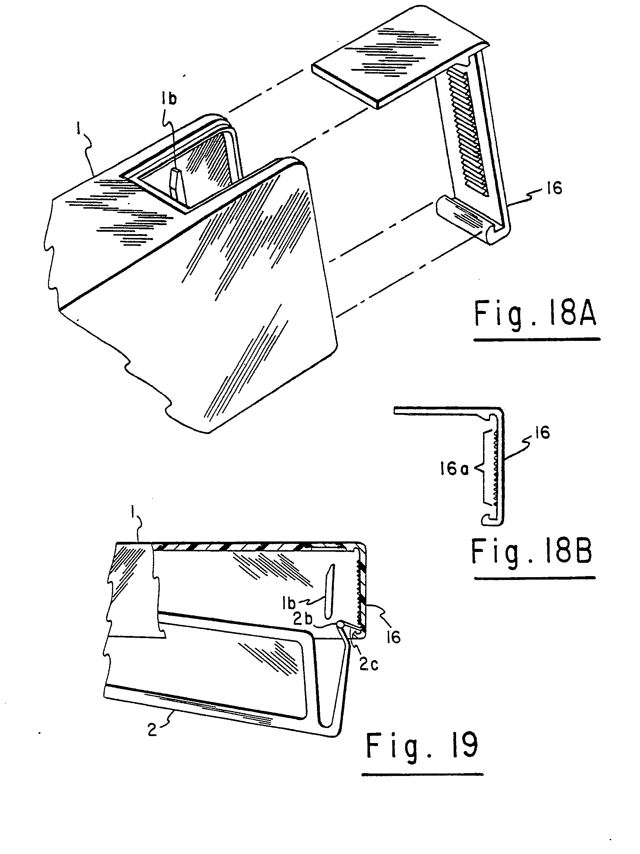

Referring to Figures 18 and 19 showing an alternative embodiment of the stapling instrument control means, to prevent partially compressing the trigger 2, partially forming a staple 12, and then allowing the trigger to return and pick up the next staple, a ratchet 16a is built into the cover 16. A stop 2c is built into the trigger 2. Cam guides 1b are built into handle 1. When the tirgger 2 is compressed, the ratchet 16a engages stop 2c and prevents the trigger from returning to its relaxed position. The trigger must be compressed past the ratchet 16a and must be completely closed so that the guide pins 2b (more fully shown in Figure 6) move up and cross over the cam guides 1b.

The guide pins 2b are spring loaded. Thus when the trigger is completely compressed, the guide pins cross over the top of the cam guides 1b. On releasing the trigger from a final compression, the ratchet is thus prevented from locking on the stop 2c.

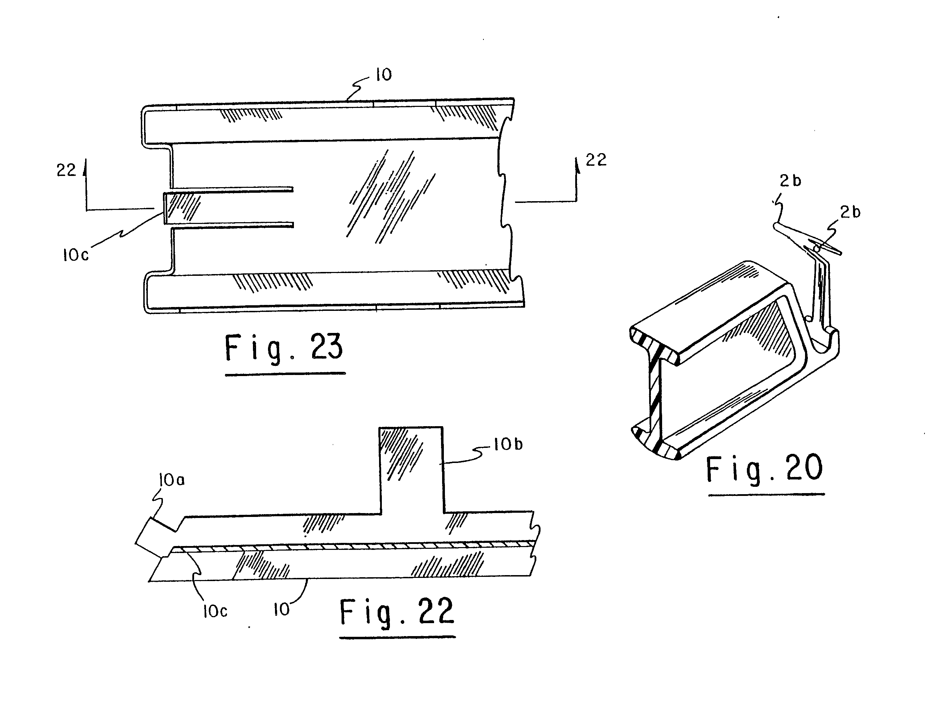

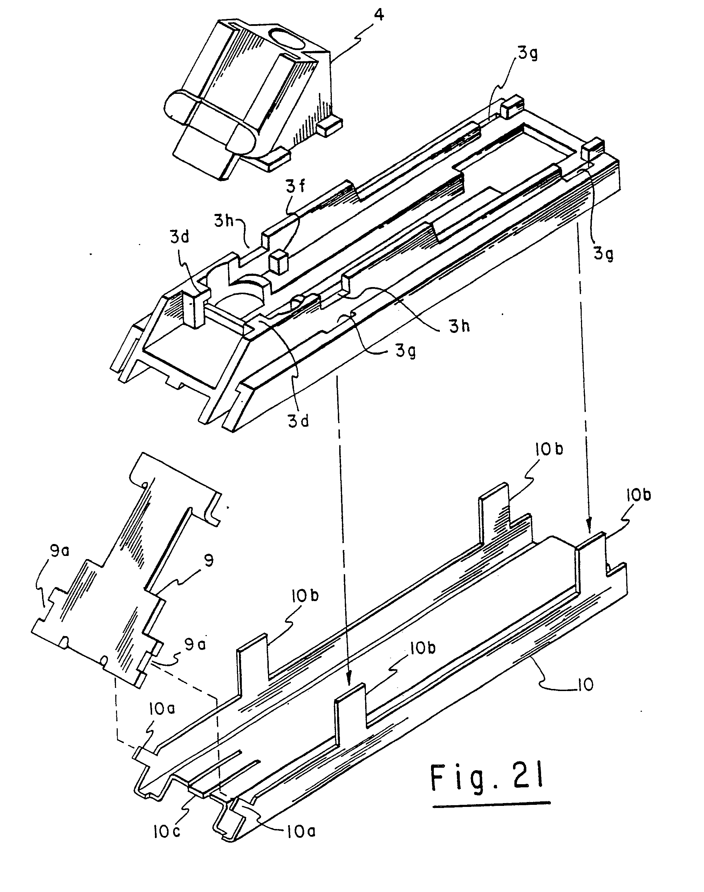

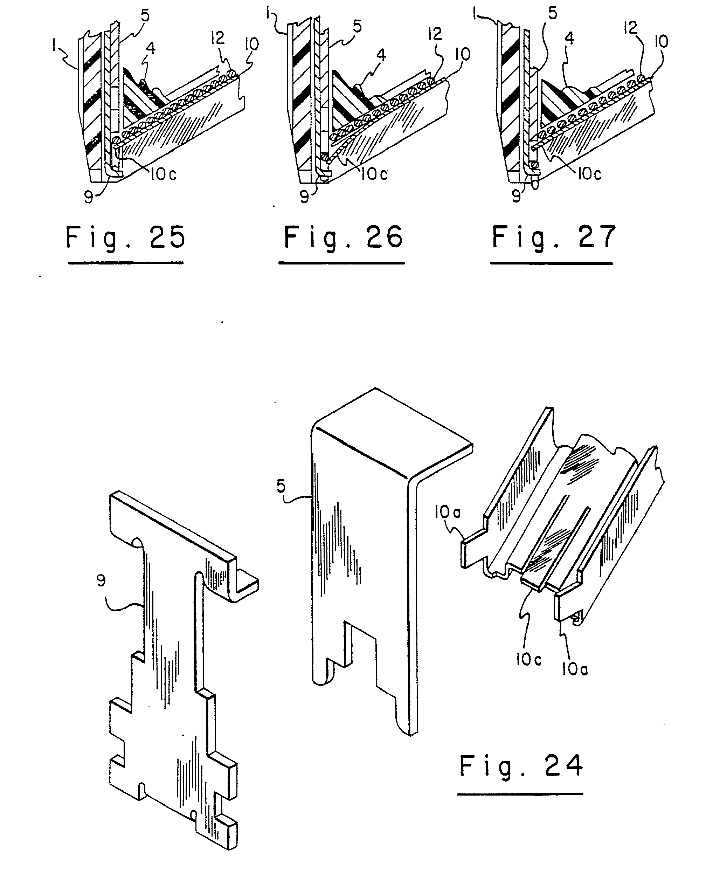

Referring to Figures 20 and 21 which show respectively, the alternative control means in the trigger and an alternative embodiment of the track and track cover, the track cover 3 is assembled as follows. The forward portion of the track cover is identical to that shown in Figures 6 to 8. The bias means 7 (not shown) and the bending anvil 9 are mounted identically to the description in Figures 6 to 8 and 17. The front tabs 10a on the track 10 pass through the slots 9a and then fold onto the bending anvil 9. As shown more fully in Figure 24, the front tabs 10a and the slots 9a are sufficiently wide to allow the forming blade 5 to move. The terminal end 13a of indicator 13 (not shown) is mounted identically to the description in Figures 6 to 10.

The guide block 4 is mounted under tabs 3d as shown in Figures 6 to 8 and 17. Blocks 3f are adjacent slots 3h on the track cover 3. Blocks 3f diagonally support the guide block 4 on the track cover 3. Forward vertical tabs 10b on the track 10 pass through the forward openings 3g in the track cover 3 and then fold onto the portion of the guide block 4 in slots 3h to hold the guide block on the track cover. Rear vertical tabs 10b pass through the rear openings 3g and then fold onto the track cover 3.

The second bias means 6 and the forming blade 5 are mounted identically to the description in Figures 6 & 17.

Figures 22 and 23 show an alternative embodiment of the track 10. In the alternative embodiment, the track 10 contains a leaf spring 10c on the terminal end of the track. The leaf spring separates the staple adjacent to the anvil surface from the anvil flange. The staples 12, staple advancing means 11, and indicator 13 are carried on the track 10 and are identical to the description in Figures 9 and 10. Referring to Figures 22 to 27, the stapling instrument with the alternative embodiments is used by placing the anvil surface 9 adjacent a wound opening or between skin or fascia. The trigger 2 (shown in Figure 1) is then compressed into the handle 1. The front end of trigger 2 engages the top flange of forming blade 5, forcing it down thru the guides 4b (shown in Figure 17) on guide block 4. The lower edges of the forming blade have a recessed area to engage staple 12. The staple is pushed downward and displaces the leaf spring 10c. The leaf spring 10c then moves back to its initial position to hold the next staple at the terminal end of track 10 and adjacent the anvil surface. The forming blade 5 continues to engage staple 12 which is then forced to bend at right angles on either side of the lower flange of anvil 9.

In the initial or rest position, the staple 12 is adjacent the vertical surface of the anvil 9, as shown in Figure 25. The forming blade 5 lowers and pushes the staple downward and onto the anvil flange. By releasing the trigger and advancing the instrument, the staple 12 is separated from the anvil flange.

**Claims**

1. A surgical stapling instrument comprising a handle (1), a trigger (2), a track (10) for carrying at least one staple (12), a blade (5) for pushing said staple (12) against an anvil (9) for closing said staple (12) and a leaf spring (10c; 15) located at the end of said track (10) and retaining said staple (12) prior to actuating the trigger (2) characterized in that the leaf spring (10; 15) is displaced by said staple (12) being pushed by said blade (5) upon actuating the trigger (2), whereby the staple (12) moves past the spring (10c; 15) towards the anvil (9) and then the spring (10c; 15) returns to its initial position.

2. A stapling instrument according to claim 1, characterized in that the trigger (2) is pivotably attached and compressible into said handle (1), the forward position of said handle (1) containing the track (10), the leaf spring (10c) provided upon the terminal end of said track (10), a portion of said leaf spring (10c) extending beyond said track (10);

a plurality of staples (12) and a staple advancing means (11) carried on said track (10);

a track cover (3) mounted on said track (10);

the anvil (9) and a first bias means (7) mounted on the cover (3), the first bias means (7) biasing the staple advancing means (11) to advance the staples (12) towards the anvil (9), said anvil (9) terminating in a perpendicular flange;

the proximal staple, from said plurality of staples (12) adjacent said anvil (9) being separated from said flange by said leaf spring (10c) biasing the underside of the staple (12) when the trigger (2) is not actuated;

a guide block (4) mounted on said cover (3) adjacent said anvil (9) and the blade (5) and a second bias means (6) movably mounted on said guide block (4), the front end of said trigger (2) engaging said blade (5) such that on compressing said trigger (2) into said handle (1), said forming blade pushes said staple (12) downward, said staple (12) displaces said leaf spring (10c), said leaf spring (10c) moves back to its initial position, and said blade (5) forms said staple (12) on said flange; and on releasing said trigger (2) and advancing said instrument said formed staple (12) is separated from said flange said second bias means (7) returns said trigger (2) and said blade (15) to their initial rest positions, and said first bias means (7) activates said staple advancing means (11) to place the forwardmost staple (12) adjacent said anvil (9).

3. A stapling instrument according to claims 1 or 2, characterized in that a flexible ratchet arm (2a) is formed on the rearward portion of said trigger (2); at least one guide pin (2b) is attached to one end of said ratchet arm (2a); a non-pivoting pawl (14a) is attached to the rearward portion of said handle (1) to cooperate with said ratchet arm (2a); and guide means (1b) having upper and lower portions, are positioned adjacent the rearward portion of said handle (1) so as to cooperate with and provide tension on said guide pin (2b), such that on partially compressing said trigger (2) the lower portion of said guide means (1b) provides tension on said guide pin (2b) and flexes said ratchet arm (2a) into engagement with said pawl (14a) and on complete compression of said trigger (2), the upper portion of said guide means (11) releases tension from said guide pin (2b) allowing said ratchet arm (2a) to relax and allowing said guide pin (2b) to cross over said guide means (1b), thus causing said ratchet arm (2a) to be disengaged from said pawl (14a).

4. A stapling instrument according to claims 1 or 2, characterized in that a flexible arm is mounted on the rearward portion of said trigger (2); a pawl (2c) and at least one guide pin (2b) are attached to one end of said arm; a ratchet (16a) is attached to the rearward portion of said handle (1); and guide means (1b) having upper and lower portions is positioned adjacent the rearward portion of said handle (1) so as to cooperate with and provide tension to said guide pin (2b), such that on partially compressing said trigger (2) the lower portion of said guide means (1b) provides tension on said guide pin (2b) and flexes said arm such that the pawl (2c) engages said ratchet (16a) and such that on complete compression of said trigger (2), the upper portion of said guide means (1b) releases tension from said guide pin (2b) allowing said arm to relax and allowing said guide pin (2b) to cross over said guide means (1b), thus causing said pawl (2c) to be disengaged from said ratchet (16a).

5. A stapling instrument according to claims 3 or 4, characterized in that said guide means (1b) are two cams attached to each side of said handle (11).

6. A stapling instrument according to claim 5, characterized in that two guide pins (2b) cooperate with said cams (1b).

7. A stapling instrument according to one of claims 1 to 5, characterized by an indicator (13) having an initial end visible in said handle (1) and a terminal end movably mounted in said advancing means (11) such that on releasing said trigger (2), said first bias means (7) pulls the terminal end and thus moves the initial end of said indicator (13) to indicate the number of staples (12) remaining.

8. A stapling instrument according to one of claims 2 to 7, characterized in that said flange is oriented opposite to the direction of stapling.

## Revendications

1. Instrument de pose d'agrafes chirurgicales comprenant une poignée (1), une gâchette (2), une glissière (10) pour transporter au moins une agrafe (12), une lame (5) pour pousser ladite agrafe (12) contre une platine (9) afin de fermer ladite agrafe (12) et un ressort lame (10c; 15) placé à l'extrémité de ladite glissière (10) et retenir lesdites agrafes (12) avant l'actionnement de la gâchette (2), caractérisé en ce que le ressort lame (10c; 15) est écarté par ladite agrafe (12) lorsque celle-ci est poussée par ladite lame (5) à la suite de l'actionnement de la gâchette (2), de sorte que l'agrafe (12) franchit le ressort (10c; 15) en se dirigeant vers la platine (9) et que le ressort (10c; 15) revient à sa position initiale.

2. Instrument de pose d'agrafes selon la revendication 1, caractérisé en ce que la gâchette (2) est articulée sur ladite poignée (1) et peut être enfoncée dans ladite poignée, la partie avant de ladite poignée (1) contenant la glissière (10), le ressort lame (10c) prévu sur l'extrémité terminale de ladite glissière (10), une partie dudit ressort lame (10c) s'étendant au-dela de ladite glissière (10);

une pluralité d'agrafes (12) et des moyens (11) d'avance des agrafes portés par ladite glissière (10); un couvre-glissière (3) monté sur ladite glissière (10); la platine (9) et des premiers moyens de sollicitation (7) montés sur le couvre-glissière (3), les premiers moyens de sollicitation (7) sollicitant les moyens (11) d'avance des agrafes pour faire avancer les agrafes (12) vers la platine (9), ladite platine (9) se terminant par un talon perpendiculaire;

l'agrafe proximale de ladite pluralité d'agrafes (12), qui est adjacente à ladite platine (9) étant séparée dudit talon par ledit ressort lame (12c) qui sollicite la face inférieure de l'agrafe (12) lorsque la gâchette (2) n'est pas actionnée;

un bloc de guidage (4) monté sur ledit couvre-glissière (3) dans la région adjacente à ladite platine (9); et la lame (5) et des deuxièmes moyens de sollicitation (6) montés mobiles sur ledit bloc de guidage, l'extrémité avant de ladite gâchette (2) attaquant ladite lame (5) de sorte que, lorsqu'on enfonce ladite gâchette (2) dans ladite poignée (1), ladite lame de mise en forme pousse ladite agrafe (12) vers le bas, ladite agrafe (12) écarte ledit ressort lame (10c), ledit ressort lame (10c) revient à sa position initiale, et ladite lame (5) met ladite agrafe (12) en forme sur ledit talon et que, lorsqu'on relâche ladite gâchette (2) et qu'on avance ledit instrument, ladite agrafe (12) mise en forme est séparée dudit talon lesdits deuxièmes moyens de sollicitation (7) ramènent ladite gâchette (2) et ladite lame (15) à leurs positions de repos initiales, et lesdits premiers moyens de sollicitation (7) activent lesdits moyens (11) d'avance des agrafes pour placer l'agrafe extrême avant (12) dans la région adjacente à ladite platine (9).

3. Instrument de pose d'agrafes selon l'une des revendications 1 et 2, caractérisé en ce qu'un bras d'encliquetage flexible (2a) est formé sur la partie arrière de ladite gâchette (2); au moins une cheville de guidage (2b) est fixée à une première extrémité dudit bras d'encliquetage (2a); un cliquet non pivotant (14a) est fixé à la partie arrière de ladite poignée (1) pour coopérer avec ledit bras d'encliquetage (2a); et des moyens de guidage (1b) comprenant une partie supérieure et une partie inférieure sont positionnés adjacents à la partie arrière de ladite poignée (1) de manière à coopérer avec ladite cheville de guidage (2b) et à exercer une tension sur cette cheville de guidage de telle manière que, lorsqu'on enfonce partiellement ladite gâchette (2), la partie inférieure desdits moyens de guidage (1b) exerce une tension sur ladite cheville de guidage (2b) et fait fléchir ledit bras d'encliquetage (2a) pour le mettre en prise avec ledit cliquet (14a) et que, lorsqu'on enfonce entièrement ladite gâchette (2) la partie supérieure desdits moyens de guidage (11) libère la tension de ladite cheville de guidage (2b) en permettant audit bras d'encliquetage (2a) de se détendre et en permettant à ladite cheville de guidage (2b) de passer au-dessus desdits moyens de guidage (1b) en amenant ainsi ledit bras d'encliquetage (2a) à se dégager du cliquet (14a).

4. Instrument de pose d'agrafes selon l'une des revendications 1 et 2, caractérisé en ce qu'un bras flexible est monté sur la partie arrière de ladite gâchette (2); un cliquet (2c) et au moins une cheville de guidage (2b) sont fixés à une première extrémité dudit bras; un organe d'encliquetage (16a) est fixé à la partie arrière de ladite poignée (1); et des moyens de guidage (1b) possèdant une partie supérieure et une partie inférieure sont positionnés adjacents à la partie arrière de ladite poignée (1) de manière à coopérer avec ladite cheville de guidage et à exercer une tension sur cette cheville de guidage, de sorte que, lorsqu'on enfonce partiellement ladite gâchette (2), la partie arrière desdits moyens de guidage (1b) exerce une tension sur ladite cheville de guidage (2b) et fait fléchir ledit bras, de sorte que le cliquet (2c) attaque ledit organe d'encliquetage (16a) et de telle manière que, lorsqu'on enfonce entièrement ladite gâchette (2), la partie supérieure desdits moyens de guidage (1b) relâche la tension sur ladite cheville de guidage (2b) en laissant ledit bras se détendre et en laissant ladite cheville de guidage (2b) passer au-dessus desdits moyens de guidage (1b) en amenant ainsi ledit cliquet (2c) à se dégager dudit organe d'encliquetage (16a).

5. Instrument de pose d'agrafes selon l'une des revendications 3 et 4, caractérisé en ce que lesdits moyens de guidage (1b) sont deux cames fixées aux deux côtés de ladite poignée (11).

6. Instrument de pose d'agrafes selon la revendication 5, caractérisé en ce que les deux chevilles de guidage (2b) coopèrent avec lesdites cames (1b).

7. Instrument de pose d'agrafes selon l'une des revendications 1 à 5, caractérisé par un indicateur (13) possèdant une extrémité initiale visible dans ladite poignée (1) et une extrémité terminale qui est montée mobile dans lesdits moyens d'avance (11) de sorte que, lorsqu'on relâche ladite gâchette, lesdits premiers moyens de sollicitation tirent l'extrémité terminale et, de cette façon, déplacent l'extrémité initiale dudit indicateur (13) pour indiquer le nombre d'agrafes (12) restantes.

8. Instrument de pose d'agrafes selon l'une des revendications 2 à 7, caractérisé en ce que ledit talon est orienté en sens inverse de la direction de pose des agrafes.

**Patentansprüche**

1. Chirurgisches Klammerinstrument mit einem Handgriff (1), einem Auslöser (2), einer Führungsschiene (10) zur Förderung von mindestens einer Klammer (12), einer Schneide (5), um die Klammer (12) im Zuge des Schliessens der Klammer (12) gegen einen Amboß (9) zu drücken und mit einer Blattfeder (10c; 15), die am Ende der Führungsschiene (10) angeordnet ist und die Klammer (12) zurückhält, bis der Auslöser (2) betätigt wird, dadurch gekennzeichnet, daß die Blattfeder (10c, 15) ausgelenkt wird durch die Klammer (12), welche bei Betätigung des Auslösers (2) durch die Schneide (5) druckbeaufschlagt wird, wobei die Klammer (12) sich an der Feder (10c; 15) entlang und auf den Amboß (9) zubewegt und anschließend die Feder (10c; 15) in ihre Anfangsposition zurückkehrt.

2. Klammerinstrument nach Anspruch 1, dadurch gekennzeichnet, daß der Auslöser (2) schwenkbar befestigt ist und in den Handgriff (1) hineindrückbar ist, der vordere Abschnitt des Handgriffs (1) die Führungsschiene (10), die an dem terminalen Ende der Führungsschiene vorgesehene Blattfeder (10c) enthält, wobei ein Teil der Blattfeder (10c) sich über die Führungsschiene (10) hinaus erstreckt; eine Vielzahl von Klammern (12) sowie eine auf der Führungsschiene (10) vorgesehene Klammerfördereinrichtung (11);

eine Schienenabdeckung (3) auf der Führungsschiene (10);

den Amboß (9) und eine erste Vorspanneinrichtung (7) auf der Abdeckung (3), wobei die erste Vorspanneinrichtung (7) die Klammerfördereinrichtung (11) so beaufschlagt, daß die Klammern (12) auf den Amboß (9) vorwärtsbewegt werden, wobei der Amboß (9) in einem senkrechten Flansch endet;

wobei die nächstliegende Klammer aus der genannten Vielzahl von Klammern (12), die dem Amboß (9) benachbart ist, durch die Blattfeder (10c), welche die Unterseite der Klammer (12) beaufschlagt, wenn der Auslöser nicht betätigt wird, von dem Flansch abgetrennt wird; einen Führungsblock (4), der auf der Abdeckung (3) benachbart dem Amboß (9) angebracht ist und die Schneide (5) und eine zweite Federeinrichtung (6), die auf dem Führungsblock (4) bewegbar befestigt ist, wobei das vordere Ende des Auslösers (2) mit der Schneide (5) derart zusammenwirkt, daß beim Eindrücken des Auslösers (2) in den Handgriff (1) die Formungsschneide die Klammer (12) nach unten drückt, die Klammer (12) die Blattfeder (10c) auslenkt, die Blattfeder (10c) in ihre Anfangsposition zurückkehrt und die Schneide (5) die Klammer (12) auf dem Flansch formt; und bei Freigabe des Auslösers (2) und der Vorwärtsbewegung des Instruments die geformte Klammer (12) von dem Flansch getrennt wird, wobei die zweite Federeinrichtung (7) den Auslöser (2) und die Schneide (15) in ihre Anfangsruhepositionen zurückführt und die erste Federeinrichtung (7) die Klammerfördereinrichtung (11) aktiviert, so daß die vorderste Klammer (12) benachbart dem Amboß (9) plaziert wird.

3. Klammerinstrument nach einem der Ansprüche 1 oder 2, dadurch gekennzeichnet, daß ein flexibler Ratschenarm (2a) am hinteren Abschnitt des Auslösers (2) ausgebildet ist; mindestens ein Führungsstift (2b) an einem Ende des Ratschenarms (2a) vorgesehen ist, eine nicht schwenkbare Klaue (14a) am hinteren Ende des Handgriffs (1) in der Weise angebracht ist, daß sie mit dem Ratschenarm (2) zusammenwirkt, und Führungseinrichtungen (1b) mit oberen und unteren Abschnitten benachbart dem hinteren Bereich des Handgriffs (1) in der Weise positioniert sind, daß sie mit dem Führungsstift (2b) zusammenwirken und auf diesen eine Spannung ausüben, so daß bei einer teilweisen Kompression des Auslösers (2) der untere Abschnitt der Führungseinrichtung (1b) Spannung auf den Führungsstift (2b) ausübt und den Ratschenarm (2a) verbiegt, so daß er mit der Klaue (14a) zusammenwirkt und bei vollständiger Kompression des Auslösers (2) der obere Abschnitt der Führungseinrichtung (11) Spannung von dem Führungsstift (2b) wegnimmt, so daß der Ratschenarm (2a) sich entspannt und ermöglicht, daß der Führungsstift (2b) über die Führungseinrichtung (1b) hinweggeht, wodurch der Ratschenarm (2a) und die Klaue (14a) außer eingriff gelangen.

4. Klammerinstrument nach einem der Ansprüche 1 oder 2, dadurch gekennzeichnet, daß ein flexibler Arm am hinteren Abschnitt des Auslösers (2) angebracht ist; eine Klaue (2c) und mindestens ein Führungsstift (2b) an einem Ende des Arms angebracht sind, eine Ratsche (16a) am hinteren Abschnitt des Handgriffs (1) vorgesehen ist, und eine Führungseinrichtung (1b) mit oberen und unteren Abschnitten benachbart dem hinteren Abschnitt des Handgriffs (1) in der Weise positioniert ist, daß sie mit dem Führungsstift (2b) zusammenwirkt und diesen derart beaufschlagt, daß beim teilweisen Zusammendrücken des Auslösers (2) der untere Abschnitt der Führungseinrichtung (1b) den Führungsstift (2b) beaufschlagt und den genannten Arm derart verbiegt, daß die Klaue (2c) mit der Ratsche (16a) zusammenwirkt und daß bei vollständiger Kompression des Auslösers (2) der obere Abschnitt der Führungseinrichtung (1b) die Spannungsbeaufschlagung von dem Führungsstift (2b) wegnimmt, so daß sich der genannte Arm entspannen kann und der Führungsstift (2b) über die Führungseinrichtung (1b) hinweggehen kann, wodurch die Klaue (2c) und die Ratsche (16a) außer Eingriff gelangen.

5. Klammerinstrument nach einem der Ansprüche 3 oder 4, dadurch gekennzeichnet, daß die Führungsseinrichtung (1b) aus zwei Nocken besteht, die an jeder Seite des Handgriffs angebracht sind.

6. Klammerinstrument nach Anspruch (5), dadurch gekennzeichnet, daß zwei Führungsstifte (2b) mit den Nocken (1b) zusammenwirken.

7. Klammerinstrument nach einem der Ansprüche 1 bis 5, gekennzeichnet durch einen Indikator (13) mit einem Anfangsende, das in dem Handgriff (1) sichtbar ist, und mit einem Schlußende, das bewegbar auf der Fördereinrichtung (11) befestigt ist, derart, daß bei Freigabe des Auslösers (2) die erste Federeinrichtung (7) das terminale Ende zieht und auf diese Weise das Anfangsende des Indikators (13) bewegt, so daß die Anzahl der verbleibenden Klammern (12) angezeigt wird.

8. Klammerinstrument nach einem der Ansprüche 2 bis 7, dadurch gekennzeichnet, daß der Flansch entgegengesetzt zur Richtung des Klammervorgangs orientiert ist.

Fig.2

Fig.3

Fig.1

0 040 683

Fig.4B

14

14a

Fig.4A

1b

2b

14a

2a

14

1b

Fig.5

1

2

# Fig. 6

Fig. 8

Fig. 7

4

Fig.10

Fig.9

Fig.17

Fig. 12

Fig. 14    Fig. 15    Fig. 16

Fig. 13

Fig. 11

0 040 683

1b

1

16

**Fig. 18A**

16

16a 16

**Fig. 18B**

1

1b
2b

2c

16

2

**Fig. 19**

Fig. 23

Fig. 22

Fig. 20

**0 040 683**

Fig. 21

Fig. 25   Fig. 26   Fig. 27

Fig. 24